# EUROPEAN PATENT SPECIFICATION

(11) **EP 4 477 167 B1**
(45) Date of publication and mention of the grant of the patent: **09.12.2026**
(21) Application number: 23703627.2
(22) Date of filing: 07.02.2023
(51) Int. Cl.: A61B 17/88, A61B 17/02

(54) **DILATOR FOR SPINAL SURGERY**
DILATATOR FÜR SPINALE CHIRURGIE
DILATATEUR POUR CHIRURGIE DE LA COLONNE VERTÉBRALE

(30) Priority: 07.02.2022 EP 22382099
(43) Date of publication of application: 18.12.2024
(73) Proprietor: Hoogland Spine Products GmbH, 85774 Unterföhring (DE); Morgenstern De Muller, Christian Rudolf, 08950 Barcelona (ES); Morgenstern Lopez, Rudolf, 08950 Barcelona (ES)
(72) Inventor: MORGENSTERN DE MULLER, Christian Rudolf, 08950 ESPLUGUES DE LLOBREGAT (BARCELONA) (ES); MORGENSTERN LOPEZ, Rudolf, 08950 ESPLUGUES DE LLOBREGAT (BARCELONA) (ES)
(74) Representative: Durán-Corretjer, S.L.P.
(86) International application number: PCT/ES2023/070066
(87) International publication number: WO 2023/148423

(56) References cited:
- EP-A1- 2 911 733
- US-A1- 2018 271 513
- US-A1- 2021 186 316
- US-B1- 9 028 522

## Description

The present invention relates to a dilator device for percutaneous spinal surgery with a transforaminal approach, and more specifically to a dilator which comprises a shield for protecting nerve roots coming from the spinal cord. The present invention is of particular application to percutaneous operations with a transforaminal approach carried out using the space known as Kambin's triangle, which is positioned in the neural foramen, in order to access the intervertebral disc, including vertebral arthrodesis procedures, although the present invention is not necessarily limited to said application. The dilator of the present invention creates a space at the Kambin's triangle while protecting the exiting nerve root.

Kambin's triangle is an anatomical corridor situated in the neural foramen which can be used to access critical structures in a variety of spinal procedures. Kambin's triangle is known as a safe space for carrying out percutaneous procedures, being a clean access route to the intervertebral disc. It is considered safe because it lacks important vascular and/or neural elements. However, it has the problem that space is limited at some points (8-10 mm) and that it is delimited on one side by an exiting nerve root. In endoscopic and/or percutaneous operations there is therefore a danger of damaging the nerve root, either by involuntary cutting during a cutting operation in an articular process to create more space, or by pressure on the nerve applied by the endoscopic devices (for example, the cannula), which may cause dysaesthesia or inflammation of the nerve. This has limited the viable operations that can be done by an approach via the neural foramen.

Document EP2911733A discloses a dilator unit provided with a shield for the nerve. The object of the shield is to protect the nerve root during percutaneous operations which use the neural foramen as an access route. The unit comprises an elongate dilator shaft and a shield. The transverse cross sections of the shaft and shield are complementary and form a circle. The elongate dilator shaft has the function of placing the shield in position. In use, the unit is positioned in the disc space at the required position and rotated to a point at which the outer surface of the shield is adjacent to the nerve root. At this point, the dilator shaft may be withdrawn, leaving the shield in place. An object pursued in this document consists of allowing a box or cage to be placed in the intervertebral space which prevents damage to the nerve root.

The dilator unit of document EP2911733A has a circular transverse cross section which is formed by the dilator shaft and the shield, which are in contact with one another. The unit has at the centre of its transverse cross section an opening corresponding to a passage for receiving a guide wire or thread. The dilator shaft and the shield come in contact along two planar surfaces perpendicular to one another. The shield has recesses in each of said perpendicular planar surfaces. Corresponding projections of the dilator shaft are arranged in said recesses. The arrangement of the perpendicular surfaces and the consequent perpendicular arrangement of the recesses allows shaft and shield to slide along the longitudinal or axial axis of the unit, but any movement in another direction is prevented, and both elements are therefore interlocked. The outer surface of the shield occupies approximately 200° of the 360° of the outer circumference of the unit.

A problem with this device of document EP2911733A is that it does not leave enough space to be able to operate afterwards, as the shield significantly reduces the space once positioned. This reduction in space still limits the possibilities and, in particular, the introduction of an intervertebral box or cage is not viable. In fact, the present inventor is not aware that said device has been commercialised.

An object of the present invention is to disclose a series of means that allow dilatation of the tissues at a Kambin's triangle during percutaneous spinal surgery with a transforaminal approach having a nerve shield for protecting the nerve root to be placed without having the drawbacks of the prior art. The present invention is defined in claim 1 while preferred embodiments are set forth in the dependent claims.

In particular, the present invention discloses a dilator for percutaneous spinal surgery, preferably for creating a space at a Kambin's triangle at percutaneous spinal surgery with a transforaminal approach, which comprises a main elongate dilator body and an elongate shield for protecting the nerve root, the main dilator body and the shield each comprising mutually complementary contact surfaces and in which the main body and the shield define a peripheral surface of the dilator in such a way that main body and shield define a transverse cross section of the dilator, it being possible for the main body and the shield to slide one relative to the other along an axial axis of the dilator, the dilator also comprising a through-hole for receiving a guide wire, in which said hole is placed eccentrically with respect to a combined transverse cross section of the main body and of the shield.

The eccentric placement of the hole allows, after the removal of the main body, a guide cable to be used which had been housed in said hole in order to carry out bone cutting operations without having to move or apply pressure to the shield, which could cause damage to the nerve root. Preferably, the hole is placed eccentrically with respect to the cross section of the main body.

The present invention also discloses a dilator for percutaneous spinal surgery, preferably for creating a space at a Kambin's triangle at percutaneous spinal surgery with a transforaminal approach, which comprises a main elongate dilator body and an elongate shield for protecting the nerve root, the main dilator body and the shield each comprising mutually complementary contact surfaces and in which the main body and the shield define a peripheral surface of the dilator in such a way that main body and shield define a transverse cross section of the dilator, it being possible for the main body and the shield to slide one relative to the other along an axial axis of the dilator, the dilator also comprising a through-hole for receiving a guide rod, in which the transverse cross section of the dilator is oblong or elongate in shape, the shield being arranged on one of the elongate faces of the transverse cross section.

The elongate shape combined with the eccentric arrangement of the hole allows for a shield which protects more space and which is not inserted excessively into Kambin's triangle, as this reduces the space that is subsequently available.

The present invention also discloses a dilator for percutaneous spinal surgery, preferably for creating a space at a Kambin's triangle at percutaneous spinal surgery with a transforaminal approach, which comprises a main elongate dilator body and an elongate shield for protecting the nerve root, the main dilator body and the shield each comprising mutually complementary contact surfaces and in which the main body and the shield define a peripheral surface of the dilator in such a way that main body and shield define a transverse cross section of the dilator, it being possible for the main body and the shield to slide one relative to the other along an axial axis of the dilator, the dilator also comprising a through-hole for receiving a guide rod, in which the shield has a laminar or curved laminar shape.

The laminar shape avoids the presence of elements of the shield which are introduced into the available operating space.

Said features (eccentricity of the guide hole, oblong shape of the dilator and laminar shape of the shield) may be implemented individually in dilators, combined in pairs or all three combined together.

Preferably, the main body is elongate in transverse cross section, the shield being arranged on one of the elongate sides of the transverse cross section. Also preferably, the main body has an equal or large dimension than the shield along a main axis of the elongate or oblong section of the dilator, more preferably the main axis is the larger axis of the elongate or oblong section. More preferably, the main body has larger dimensions than the shield along two main axes of the elongate or oblong section of the dilator, said main axes being perpendicular to each other. This allows for less space being occupied by the nerve shield, which space would reduce the space that is subsequently available.

According to the present invention, the main body and shield do not need to be interlocked so as to only be able to slide in respect to one another in the axial direction. Preferably, the shield has no recesses for interlocking with the main body. This allows a slimmer shield to be obtained which therefore occupies less space. In addition, preferably, the shield also has no projections for interlocking. If it is decided to produce recesses or projections for interlocking between the main body and the shield, it is preferable to produce said recesses and projections at each end of the transverse cross section of the shield, thus leaving the central portion free and therefore affecting the thickness, which should preferably be the minimum possible, of the shield as little as possible. Preferably, the main body is monolithic in cross section, not having parts that can slide relative to each other along the longitudinal direction of the dilator.

According to the present invention, both shield and main body may have retention means to restrict - but not prevent - movement between shield and main body in a radial direction, said retention means being situated at both ends of the transverse cross section of the shield and which preferably comprise the shape of said ends which preferably have a curved shape. In this case, the retention means restrict movement by providing a resistance which can be overcome.

As it is apparent from the above, the present invention discloses a dilator which is advantageous for the dilation of tissues at a Kambin's triangle. For this reason, the dimensions of the transverse cross section of the dilator should be such that it can pass through a human Kambin's triangle. For this, preferably, in some embodiments, the transverse cross section of the dilator may be approximately 9 mm or less on a long axis and 5.5 mm or less along an axis perpendicular to said long axis.

The present invention also discloses kits of tools for performing a spinal surgery, preferably with a transforaminal approach, which comprise a dilator according to the present invention. The kit may also comprise a drill bit, or various drill bits of different diameters. The kit may also comprise a guide cable, for example a so-called "K-wire". The kit may also comprise known elements of transforaminal access systems, such as Tom Shidi needles (for example with a blunt tip, and/or a cutting tip and/or a drill) and controls for the various transcutaneous components.

The dilator according to the present invention may be used in various transcutaneous spinal operations with a transforaminal approach, including vertebral arthrodesis in general and with the installation of intervertebral box(es) or cage(s) in particular. Preferably, the dilator according to the present invention may be used in operations that involve cutting a portion of bone (typically from the articular process) to access the disc space. A typical sequence consists of inserting a guide cable, inserting the dilator along the guide cable and removing the main body of the dilator while leaving the shield. In this way the nerve root remains protected. Then additional spine surgery operations may be carried out using the space generated by the dilator, such as anyone of the group comprising bone cutting using a drill bit, cleaning and preparing the disc space and positioning and installing an intervertebral box/cage for arthrodesis, or a combination thereof, preferably in a sequential manner.

For a better understanding, drawings are attached which show, as an example, an explanatory but nonlimiting embodiment of the object of the present invention.
Fig. 1 is a perspective view of a dilator.
Fig. 2 shows the dilator from a different point of view from that shown in Fig. 1.
Fig. 3 is a medial cross section of the dilator.
Fig. 4 is another perspective view of the dilator, with the constituent parts thereof separated.
Fig. 5 to 11 show diagrammatically a method of placing an intervertebral box or cage using the dilator shown in the previous figures.
Fig. 12 shows an intervertebral box or cage.
Fig. 13 is a cross section, along a bottom plane of the box in Fig. 12.
Fig. 14 shows the fitting together of the parts that form the box and the fitting thereof in a cannula.
Fig. 15 shows one of the two parts that form the box.
Fig. 16 shows diagrammatically the position of a box or cage in an intervertebral space, after having been placed using a procedure like the one shown in Fig. 5 to 11.
Fig. 17 shows diagrammatically a commercial kit or unit which comprises a dilator like the one in Fig. 1 to 4.

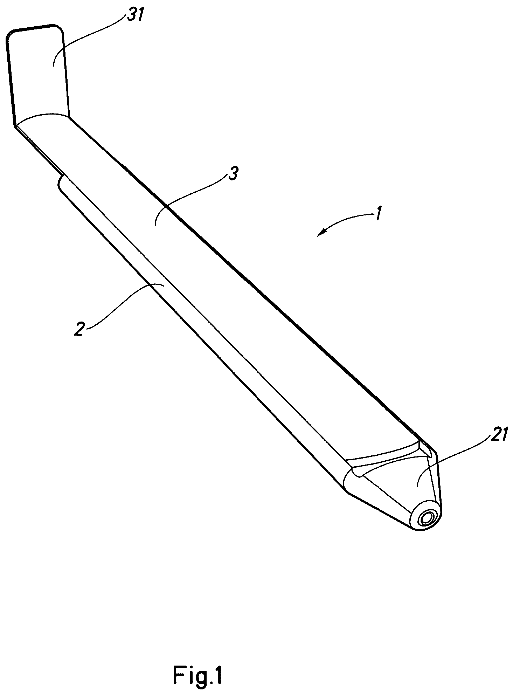

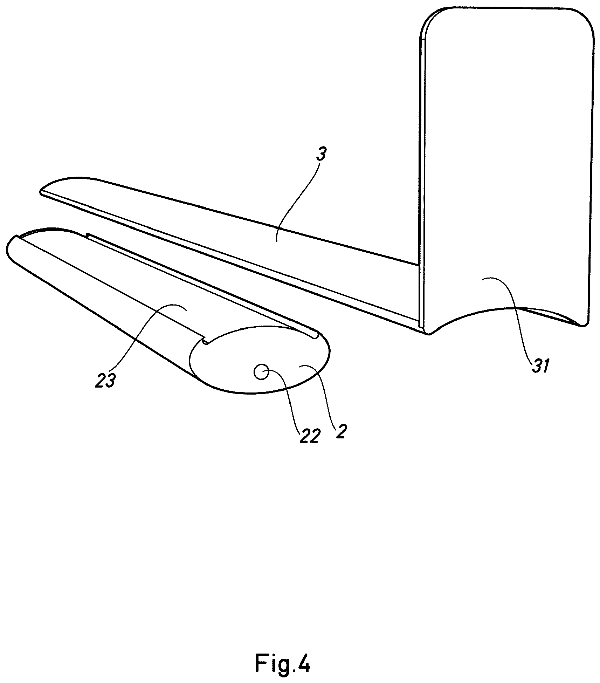

Fig. 1 to 4 show an example of a dilator 1 according to the present invention.

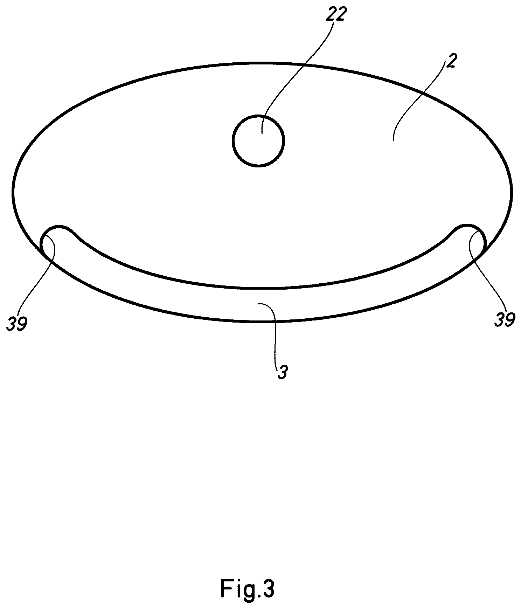

The dilator 1 is made up of a main body 2 and a shield 3. Both the main body 2 and the shield 3 have an elongate shape for use in percutaneous operations. Main body 2 and shield 3 have conjugate shapes such that the dilator 1 has a uniform outer lateral surface. More specifically, and as can be seen in Fig. 3, the cross section of the dilator 1 - made up of the cross sections of the main body 2 and the shield 3 - has an elliptical or ovoid shape. The dilator 1 also has a longitudinal through-hole 22, the object of which is to receive a guide (for example a K-wire) which will guide the trajectory of the dilator 1 inside the body of the patient. The hole 22 is situated eccentrically with respect to the centre of the cross section of the dilator. In particular, the hole 22, which is in the main body 2, is situated farther from the outer surface of the dilator 1 formed by the shield 3 than from an outer surface of the dilator 1 formed by the main body. The outer surfaces formed respectively by the main body and the shield are opposite one another. This offsetting of the hole makes it easier subsequently to create additional space by the positioning of a drill bit to cut the bone of one or both vertebrae.

The main body 2 also has a pointed distal end 21. As can be seen in Fig. 1, the main body 2 has a recess for receiving the shield, along which the shield can slide. However, it is possible for the main body and the shield not to be interlocked, so that the relative movement in a radial direction may be completely allowed, or not totally prevented.

The shield 3 has a curved laminar shape in cross section. The laminar shape helps achieve a shield that is as slender as possible. More specifically, the shield 3 is formed in cross section by two main surfaces, an outer surface which forms part of the outer surface of the dilator 1, and another inner surface parallel thereto, as well as two ends which form a connection between both main surfaces. For the shield to be more slender, both main surfaces are parallel to one another. In addition, the shield does not have attachment recesses on said main surfaces, which allows for an even more slender shield. In addition, in the case shown, neither do the ends 39 of the shield 3 have recesses for interlocking with the main body. It is not necessary to interlock the main body 2 and the shield 3 to prevent any movement between the two. If thought appropriate, the ends 39 could have projections for interlocking (not shown in the figure) or alternatively could be given a shape that restricts, but does not prevent, movement in a radial direction.

The shield may have a shank 31 for handling. The shank may take various shapes. In general, the instruments have been shown diagrammatically and any necessary connection and/or handling elements may be arranged at the distal and/or proximal ends thereof.

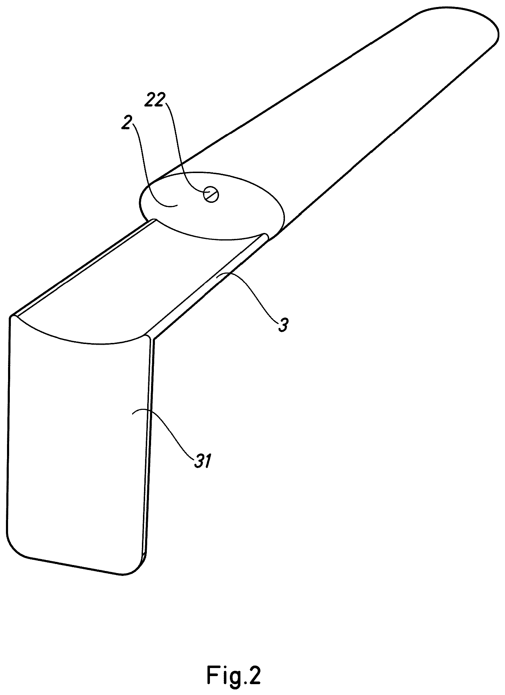

As shown diagrammatically in Fig. 2, the box is introduced using percutaneous means. An endoscopic cannula 6 has been shown in Fig. 2 which approaches the intervertebral space by the anterior route.

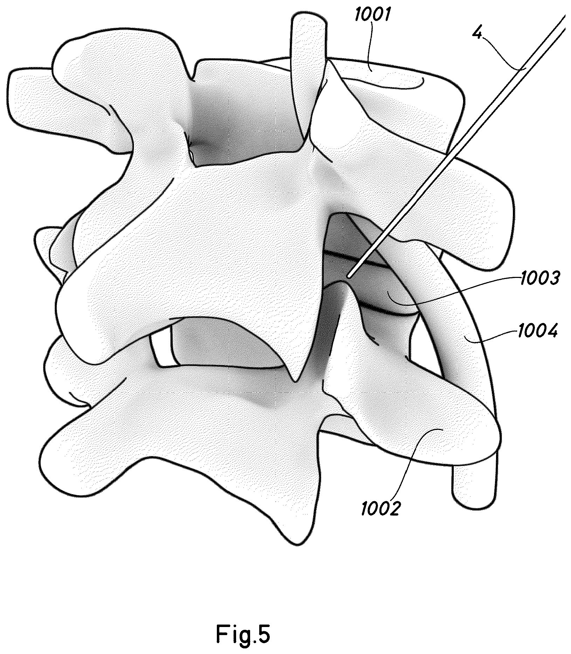

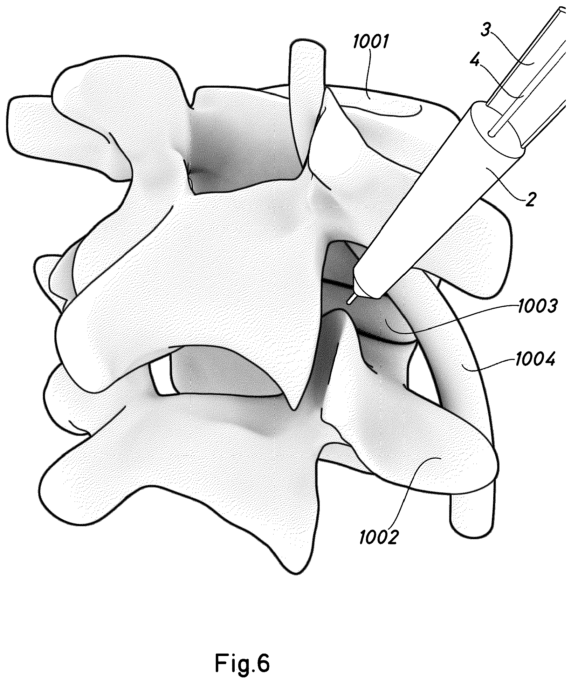

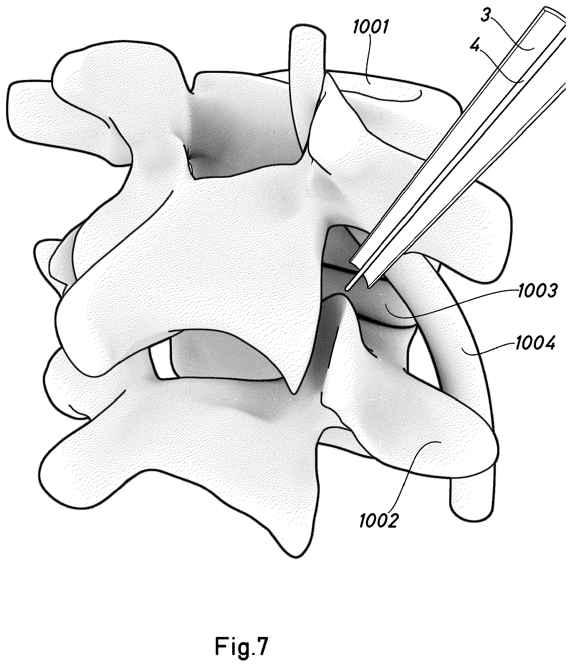

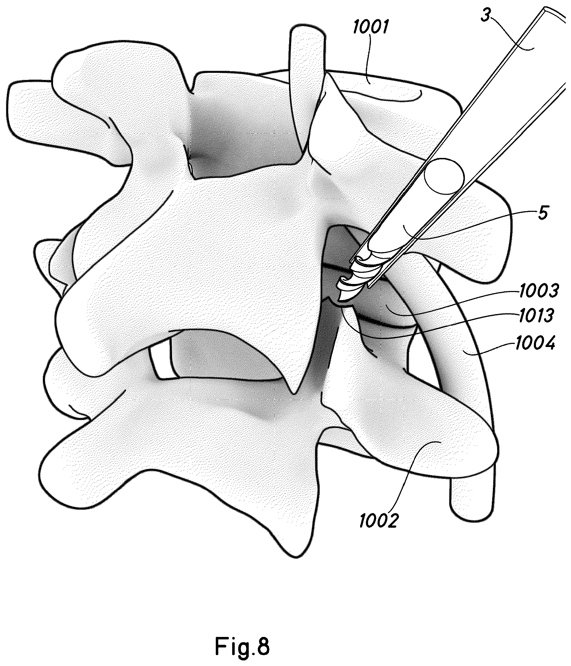

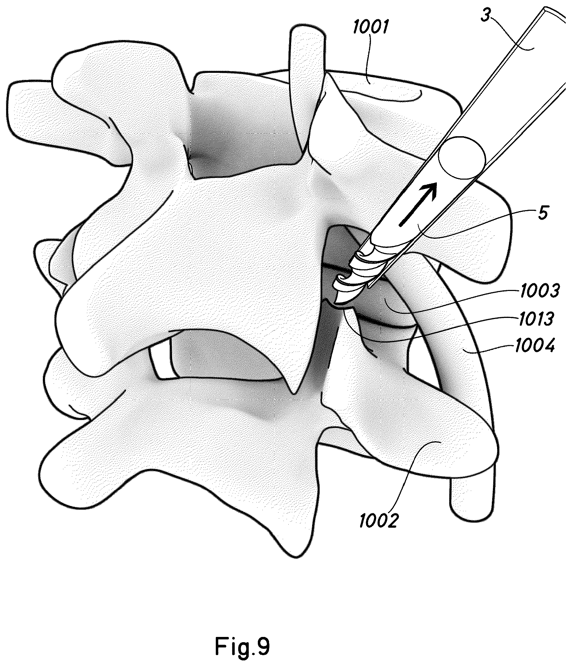

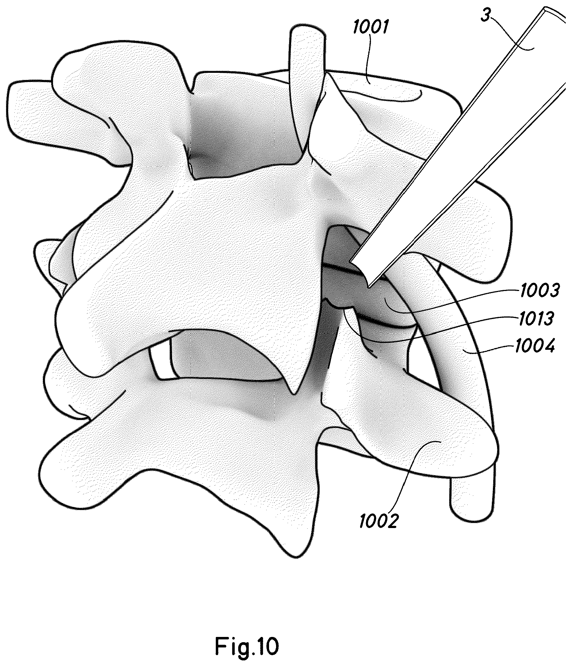

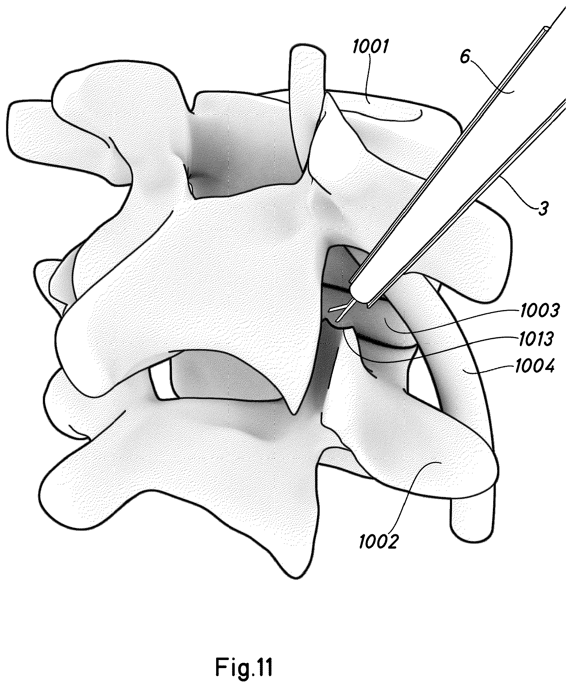

Fig. 5 to 11 show diagrammatically a procedure for placing the dilator. The method may begin (Fig. 5) by placing a guide cable 4 (K-wire) in position by a transforaminal approach. The guide cable 4 is positioned opposite the intervertebral disc 1003 situated between the vertebrae 1001, 1002 or alternatively penetrates therein. From here, said cable will serve as a guide for introducing the dilator with the corresponding shield 3 thereof facing towards the nerve root 1004 that is to be protected, as shown in Fig. 6. Next, the main body 2 of the dilator is removed, being made to slide with respect to the shield 3, which remains in position, protecting the nerve root 1004. As can be seen in Fig. 7, the guide cable 4 also remains in position. From the arrangement in Fig. 7, the required transepidermal operation by transforaminal approach may begin. Fig. 8 to 11 show a possible operation. To illustrate the operations, the endoscopic cannula that would protect the elements that are introduced, depending on what is required in each case, has not been shown in the figures. Fig. 8 shows how a drill bit 5 is introduced in order to cut part of the inferior vertebra 1002. It would also be possible to cut part of the superior vertebra, or both vertebrae, depending on requirements. The object of cutting is to gain space for subsequent operations. Since, in order to make the cut, the drill bit has to project from the surrounding cannula (not shown in the figures), the shield 3 acts as a barrier which prevents cutting and/or compression of the nerve root 1004. Fig. 8 shows the cut portion 1013. In this case, the cut portion corresponds to the superior articular process of the inferior vertebra 1002. It will be noted that the fact that the hole in the dilator is in an eccentric position with respect to the centre of the cross section of the dilator facilitates being able to cut part of the vertebra without having to move the shield 3 from its position, as this would result in applying pressure to the nerve root 1004 and causing dysaesthesia.

The drill bit 5 is then removed and it is now possible to carry out the necessary operations. If a considerable amount of bone has to be removed, it is possible to introduce two or more drill bits sequentially using an increasingly large drill bit diameter.

It is then possible to introduce a tool 6 (clamp and/or cutter, extractor, etc.) to clean the interior of the disc 1003, which leaves the way clear to place a cage or box 9 to carry out intervertebral fusion. Using this system, it is possible to introduce percutaneously various types of intervertebral boxes or cages, the object of which is to fuse and/or separate two vertebrae by being interposed in the intervertebral space.

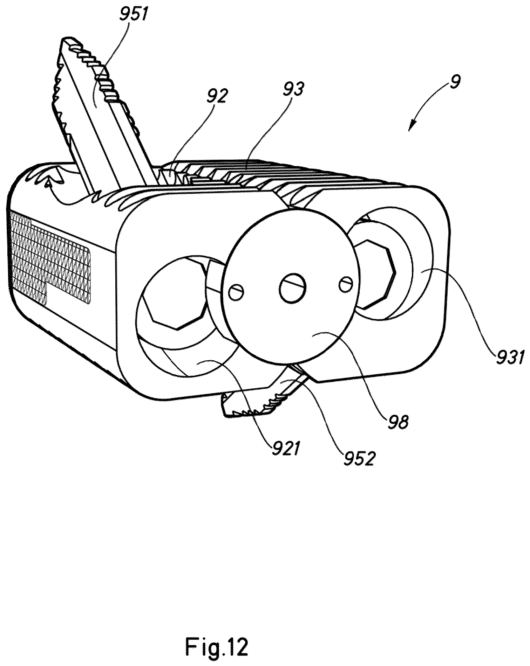

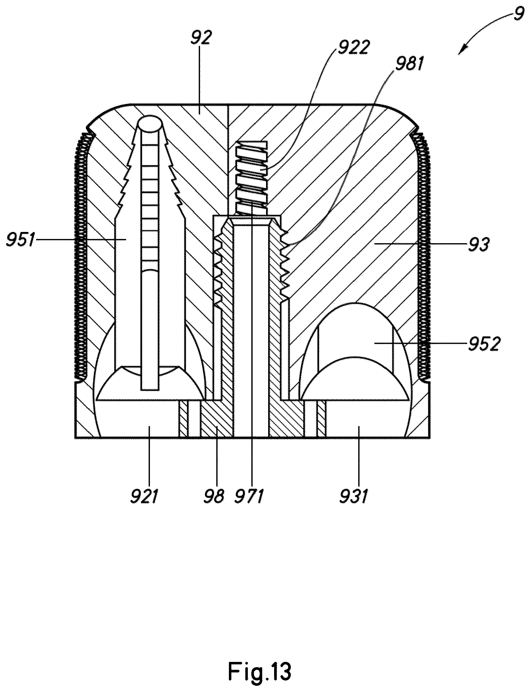

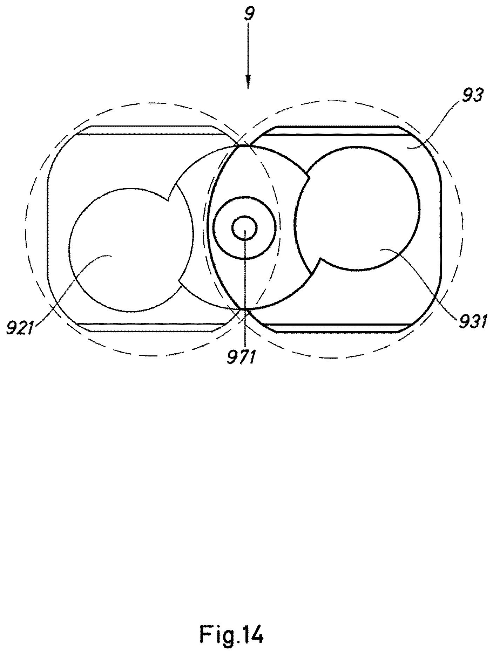

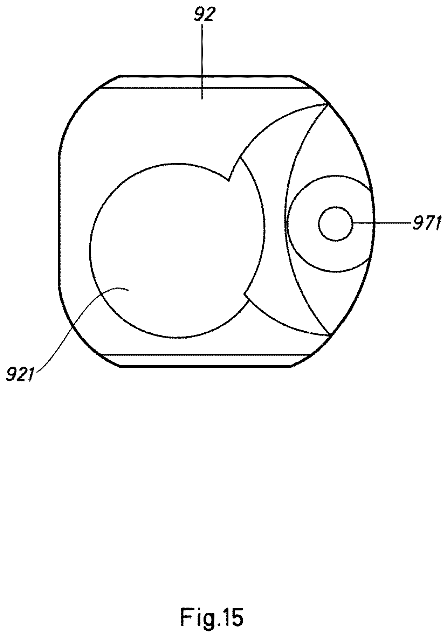

Fig. 12 to 15 show an example of a box 9 which is made up of two bodies 92, 93 having conjugate shapes. Each of the two bodies 92, 93 is of a suitable size to enter through an endoscopic cannula which passes through Kambin's triangle, for which said body has a corresponding hole 971 for a guide cable. The parts are assembled together.

When the parts are assembled, the guide holes are coaxial, one hole being a through-hole and the other having a thread 922 for receiving the endoscopic guide.

Assembly may be carried out, for example, by means of a set of respective conjugate recesses and projections situated at the contact interface between both parts. Said projections may preferably be perpendicular to the coaxial axis of said holes. Preferably, said holes are arranged on each of said conjugate projections.

The box also comprises a connection element in the form of a screw 971 which is fixed by means of a thread 981, securing the parts 92, 93. The thread 981 may be fitted into respective threads in one or both parts 92, 93. Each of the two parts 92, 93 has a through-hole 921, 931 which has an inlet in the proximal area and an outlet in one of the bases for receiving a means of fixing to the adjacent vertebrae, for example, the screws or pins 951, 952, which have sharp projections. Each of the sharp pins 951, 952 is arranged on one of the bases of the box, pointing in different directions.

The method (not claimed) of positioning the box 9 may be as follows:
First, a part 92 is positioned, being guided by an endoscopic guide which is threaded onto the corresponding thread 922 of the part 92. Without removing the guide, the other part is positioned through the endoscopic cannula, said part also being guided by the endoscopic guide. This movement causes both parts to be held against one another, interlocking owing to the conjugate shapes thereof.

Next, the pins 951, 952 are inserted, driving said pins into the adjacent vertebrae.

Finally, the screw 98 is slid along the endoscopic guide and then a tool is introduced to thread the screw 98.

Next the threading tool, the endoscopic guide, which is unscrewed, the endoscopic cannula and the shield 3 are removed.

The installation method (not claimed) may be different from the one shown, and it is also possible to use different percutaneous and even non-percutaneous techniques. The order of the operations is also susceptible to changes. The introduction route is through Kambin's triangle made safe by the use of a shield for protecting the nerve root, which is preferably positioned by means of a dilator as shown above.

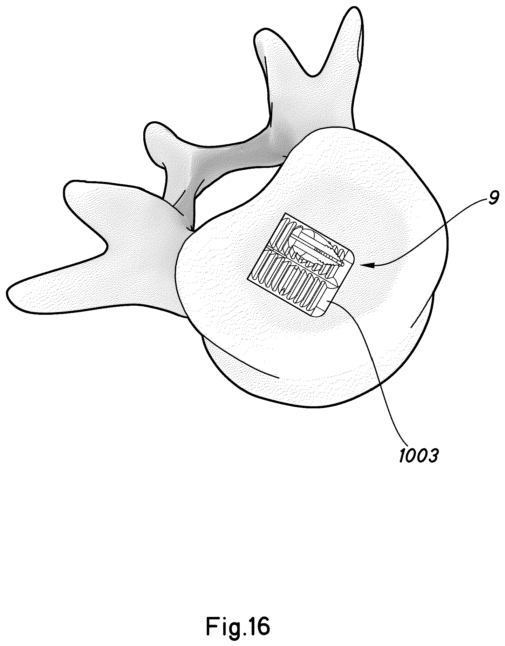

Fig. 16 shows how the box 9 would be positioned, typically at an angle owing to the transforaminal approach used for introduction thereof. However, in an intervertebral fusion operation typically two boxes 9 will be placed, one through the left neural foramen and the other through the right.

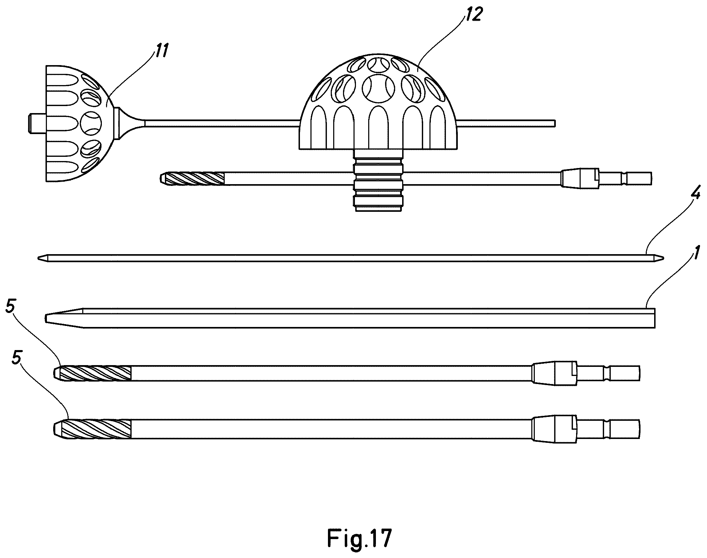

Fig. 17 shows an example of a marketable kit for carrying out transcutaneous operations which includes a dilator 1 according to the present invention, a guide cable 4 and at least one drill bit 5, or a set of drill bits of different sizes. The kit may also comprise known elements of transforaminal access systems, such as Tom Shidi needles 11 (for example with a blunt tip, and/or a cutting tip and/or a drill and controls 12 for the various elements, etc.). In general, the heads for connection to the control of the different elements have not been shown in the figures so as not to complicate the illustrations. Nor has the use of the different endoscopic cannulas, employed as required and according to the judgement of the specialist doctor, been illustrated.

The dimensions of the dilator shown may vary depending on the type of vertebra to which said dilator will be directed. In the example, the transverse cross section of the dilator may be approximately 9 mm on the long axis and 5.5 mm along an axis perpendicular to said long axis. The shield may, for example, have a constant thickness of 0.75 mm and a length of 8 mm measured in the direction of said long axis. The through-hole of the dilator may be approximately 1.75 mm and may be arranged at a distance of at least 2 mm from the shield. The length of the dilator in the axial direction may be approximately 180 mm, and that of the shield, 170 mm.

The installation method (not claimed) may be different from the one shown, and it is also possible to use different percutaneous techniques and modifications to the one shown. The order of the operations is also susceptible to changes.

Although the invention has been described with respect to preferred embodiments, these should not be considered to limit the invention, which will be defined by the widest interpretation of the following claims.

## Claims

1. Dilator for percutaneous spinal surgery with a transforaminal approach which comprises a main elongate dilator body (2) and an elongate nerve shield (3) for protecting the nerve root, the main dilator body and the shield each comprising mutually complementary contact surfaces and in which the main body and the shield define a transverse cross section of the dilator, it being possible for the main body and the shield to slide one relative to the other along an axial axis of the dilator, the dilator also comprising a through-hole (22) for receiving a guide rod, said hole being placed eccentrically with respect to a combined transverse cross section of the main body and of the shield **characterised in that** the main body has an equal or larger dimension than the shield along a main axis of the elongate or oblong section of the dilator.

2. Dilator according to the preceding claim, **characterised in that** the transverse cross section of the dilator is oblong or elongate in shape, the shield being arranged on one of the elongate faces of the transverse cross section.

3. Dilator according to either one of the preceding claims, **characterised in that** the main body has larger dimensions than the shield along two main axes of the elongate or oblong section of the dilator, said main axes being perpendicular to each other.

4. Dilator according to any one of the preceding claims, **characterised in that** the shield has a laminar or curved laminar shape.

5. Dilator according to any one of the preceding claims, **characterised in that** the transverse cross section of the shield has a central area of constant thickness.

6. Dilator according to any one of the preceding claims, **characterised in that** the shield has no recesses for interlocking with the main body.

7. Dilator according to any one of the preceding claims, **characterised in that** the shield has no projections for interlocking with the main body.

8. Dilator according to any one of the preceding claims, **characterised in that** both shield and main body have retention means to restrict the relative movement between the main body and the shield, the retention means of the shield being situated at both ends of the transverse cross section of the shield.

9. Dilator according to the preceding claim, **characterised in that** said retention means of the shield comprise the shape of both ends which have a curved shape.

10. Dilator according to the preceding claim, **characterised in that** said projecting faces have a curved shape.

11. Dilator according to any one of the claims 2 to 10, **characterised in that** said dilator has an elliptical or ovoid shape.

12. Dilator according to any one of the preceding claims, **characterised in that** the hole (22) is placed eccentrically with respect to the cross section of the main body.

13. Dilator according to any one of the preceding claims, **characterised in that** the dimensions of the transverse cross section of said dilator can pass through a human Kambin's triangle.

14. Kit of tools for percutaneous spinal operations by a transforaminal approach, **characterised in that** it comprises a dilator according to any one of the preceding claims, a drill bit and a guide cable.

## Patentansprüche

1. Dilatator für eine perkutane spinale Chirurgie mit einem transforaminalen Ansatz, der einen länglichen Dilatator-Hauptkörper (2) und einen länglichen Nervenschutz (3) für das Schützen der Nervenwurzel umfasst, wobei der Dilatator-Hauptkörper und der Schutz jeweils komplementäre Kontaktflächen aufweisen und wobei der Hauptkörper und der Schutz einen transversalen Querschnitt des Dilatators definieren, wobei der Hauptkörper und der Schutz relativ zueinander entlang einer axialen Achse des Dilatators gleiten können, wobei der Dilatator auch ein Durchgangsloch (22) für das Aufnehmen einer Führungsstange aufweist, wobei das Loch exzentrisch in Bezug auf einen kombinierten transversalen Querschnitt des Hauptkörpers und des Schutzes ist, **dadurch gekennzeichnet, dass** der Hauptkörper eine gleiche oder größere Dimension als der Schutz entlang einer Hauptachse des länglichen oder gestreckten Schnitts des Dilatators aufweist.

2. Dilatator nach dem vorstehenden Anspruch, **dadurch gekennzeichnet, dass** der transversale Querschnitt des Dilatators eine gestreckte oder längliche Form aufweist, wobei der Schutz an einer der länglichen Flächen des transversalen Querschnitts angeordnet ist.

3. Dilatator nach einem der vorstehenden Ansprüche, **dadurch gekennzeichnet, dass** der Hauptkörper entlang von zwei Hauptachsen des länglichen oder gestreckten Schnitts des Dilatators größere Dimensionen aufweist als der Schutz, wobei die Hauptachsen senkrecht zueinander sind.

4. Dilatator nach einem der vorstehenden Ansprüche, **dadurch gekennzeichnet, dass** der Schutz eine laminare oder eine gekrümmte laminare Form aufweist.

5. Dilatator nach einem der vorstehenden Ansprüche, **dadurch gekennzeichnet, dass** der transversale Querschnitt des Schutzes einen zentralen Bereich mit einer konstanten Dicke aufweist.

6. Dilatator nach einem der vorstehenden Ansprüche, **dadurch gekennzeichnet, dass** der Schutz keine Vertiefungen für ein Eingreifen mit dem Hauptkörper aufweist.

7. Dilatator nach einem der vorstehenden Ansprüche, **dadurch gekennzeichnet, dass** der Schutz keine Vorsprünge für ein Eingreifen mit dem Hauptkörper aufweist.

8. Dilatator nach einem der vorstehenden Ansprüche, **dadurch gekennzeichnet, dass** der Schutz und der Hauptkörper Halteeinrichtungen für ein Beschränken der relativen Bewegung zwischen dem Hauptkörper und dem Schutz aufweisen, wobei die Halteeinrichtungen des Schutzes an beiden Enden des transversalen Querschnitts des Schutzes angeordnet sind.

9. Dilatator nach dem vorstehenden Anspruch, **dadurch gekennzeichnet, dass** die Halteeinrichtungen des Schutzes eine Form beider Enden mit einer gekrümmten Form aufweisen.

10. Dilatator nach dem vorstehenden Anspruch, **dadurch gekennzeichnet, dass** die Vorsprungsflächen eine gekrümmte Form aufweisen.

11. Dilatator nach einem der Ansprüche 2 bis 10, **dadurch gekennzeichnet, dass** der Dilatator eine elliptische oder ovale Form aufweist.

12. Dilatator nach einem der vorstehenden Ansprüche, **dadurch gekennzeichnet, dass** das Loch (22) exzentrisch in Bezug auf den Querschnitt des Hauptkörpers platziert ist.

13. Dilatator nach einem der vorstehenden Ansprüche, **dadurch gekennzeichnet, dass** die Dimensionen des transversalen Querschnitts des Dilatators durch das menschliche Kambin-Dreieck passen.

14. Satz von Werkzeugen für perkutane spinale Operationen mit einem transforaminalen Ansatz, **dadurch gekennzeichnet, dass** der Satz von Werkzeugen einen Dilatator gemäß einem der vorstehenden Ansprüche, einen Bohrer und ein Führungskabel umfasst.

## Revendications

1. Dilatateur pour la chirurgie percutanée de la colonne avec approche transforaminale, qui comprend un corps principal (2) de dilatateur de forme allongée et un bouclier (3) de nerf allongé pour la protection de la racine nerveuse, le corps de dilatateur principal et le bouclier comportant chacun des surfaces de contact mutuellement complémentaires et dans lequel le corps principal et le bouclier définissent une section transversale du dilatateur, ledit corps principal et ledit bouclier pouvant coulisser l'un par rapport à l'autre suivant un axe axial du dilatateur, le dilatateur comprenant également un trou traversant (22) destiné à recevoir une tige de guidage, ledit trou étant placé de manière excentrée par rapport à une section transversale combinée du corps principal et du bouclier, **caractérisé en ce que** le corps principal présente une dimension égale ou supérieure à celle du bouclier le long d'un axe principal de la section allongée ou oblongue du dilatateur.

2. Dilatateur selon la revendication précédente, **caractérisé en ce que** la section transversale du dilatateur est de forme oblongue ou allongée, le bouclier étant disposé sur l'une des faces allongées de la section transversale.

3. Dilatateur selon l'une ou l'autre des revendications précédentes, **caractérisé en ce que** le corps principal présente des dimensions supérieures à celles du bouclier le long de deux axes principaux de la section allongée ou oblongue du dilatateur, lesdits axes principaux étant perpendiculaires l'un à l'autre.

4. Dilatateur selon l'une quelconque des revendications précédentes, **caractérisé en ce que** le bouclier présente une forme laminaire ou laminaire courbe.

5. Dilatateur selon l'une quelconque des revendications précédentes, **caractérisé en ce que** la section transversale du bouclier présente une zone centrale d'épaisseur constante.

6. Dilatateur selon l'une quelconque des revendications précédentes, **caractérisé en ce que** le bouclier ne présente aucun évidement destiné à s'emboîter avec le corps principal.

7. Dilatateur selon l'une quelconque des revendications précédentes, **caractérisé en ce que** le bouclier ne présente aucune saillie destinée à s'emboîter avec le corps principal.

8. Dilatateur selon l'une quelconque des revendications précédentes, **caractérisé en ce que** le bouclier et le corps principal présentent tous deux des moyens de retenue destinés à limiter le mouvement relatif entre le corps principal et le bouclier, les moyens de retenue du bouclier étant situés aux deux extrémités de la section transversale du bouclier.

9. Dilatateur selon la revendication précédente, **caractérisé en ce que** lesdits moyens de retenue du bouclier comprennent la forme des deux extrémités, qui présentent une forme courbe.

10. Dilatateur selon la revendication précédente, **caractérisé en ce que** lesdites faces en saillie présentent une forme courbe.

11. Dilatateur selon l'une quelconque des revendications 2 à 10, **caractérisé en ce que** ledit dilatateur présente une forme elliptique ou ovoïde.

12. Dilatateur selon l'une quelconque des revendications précédentes, **caractérisé en ce que** le trou (22) est placé de manière excentrée par rapport à la section transversale du corps principal.

13. Dilatateur selon l'une quelconque des revendications précédentes, **caractérisé en ce que** les dimensions de la section transversale dudit dilatateur permettent de passer à travers un triangle de Kambin humain.

14. Kit d'outils pour interventions chirurgicales percutanées de la colonne par voie transforaminale, **caractérisé en ce qu'**il comprend un dilatateur selon l'une quelconque des revendications précédentes, un foret et un câble de guidage.
